# EUROPÄISCHE PATENTSCHRIFT

(11) **EP 3 891 116 B1**
(45) Veröffentlichungstag und Bekanntmachung des Hinweises auf die Patenterteilung: **26.10.2022**
(21) Anmeldenummer: 19809100.1
(22) Anmeldetag: 02.12.2019
(51) Int. Cl.: C07C 37/50, C07C 39/04, C08G 64/06

(54) **VERFAHREN ZUR HERSTELLUNG EINER HYDROXYVERBINDUNG DURCH DECARBOXYLIERUNG**
METHOD FOR PRODUCTION OF A HYDROXY COMPOUND BY MEANS OF DECARBOXYLATION
PROCÉDÉ DE PRÉPARATION D'UN COMPOSÉ HYDROXY PAR DÉCARBOXYLATION

(30) Priorität: 07.12.2018 EP 18211039
(43) Veröffentlichungstag der Anmeldung: 13.10.2021
(73) Patentinhaber: Covestro Intellectual Property GmbH & Co. KG, 51373 Leverkusen (DE)
(72) Erfinder: BITTNER, Natalie, 50226 Frechen (DE); LANGANKE, Jens, 53894 Mechernich (DE); MEINE, Niklas, 40223 Düsseldorf (DE); HEIJL, Jan, 9160 Lokeren (BE)
(74) Vertreter: Levpat
(86) Internationale Anmeldenummer: PCT/EP2019/083215
(87) Internationale Veröffentlichungsnummer: WO 2020/114928

(56) Entgegenhaltungen:
- EP-A1- 1 277 723
- EP-A1- 2 586 767
- JP-A- 2016 023 136
- S.-F. KOELEWIJN ET AL: "Sustainable bisphenols from renewable softwood lignin feedstock for polycarbonates and cyanate ester resins", GREEN CHEMISTRY, Bd. 19, Nr. 11, 1. Januar 2017 (2017-01-01), Seiten 2561-2570, XP055590697, GB ISSN: 1463-9262, DOI: 10.1039/C7GC00776K

## Beschreibung

Die vorliegende Erfindung betrifft ein Verfahren zur Herstellung einer speziellen Hydroxyverbindung durch Decarboxylierung einer speziellen Carbonsäureverbindung oder eines Salzes dieser Carbonsäureverbindung, ein Verfahren zur Herstellung eines Diarylcarbonats, eines Bisphenols oder eines Polycarbonats, ein Diarylcarbonat oder Bisphenol, ein Polycarbonat und ein Verfahren zur Einstellung des Isotopenverhältnisses von C14 zu C12 in einem Polymer.

Phenole mit unterschiedlichen Substitutionsmustern am Aromaten stellen die Ausgangsverbindungen für unterschiedliche Monomere und damit auch für die daraus resultierenden Polymere dar. Die Herstellung solcher Phenole aus nachwachsenden Rohstoffen stellt eine große Herausforderung dar. Eine Möglichkeit zur Herstellung biobasierten Phenols bietet die direkte Fermentation von Zuckern, wie beispielsweise in der WO 2014/076113 A1 beschrieben. Phenol ist jedoch für den dort beschriebenen Mikroorganismus toxisch und auch die Abtrennung aus der wässrigen Fermentationsbrühe ist aufwendig. Hydroxybenzoesäuren wie beispielsweise 4-Hydroxybenzoesäure, 2-Hydroxybenzoesäure und 3-Hydroxybenzoesäure können ebenfalls fermentativ aus Zuckern hergestellt werden. Da sie für die eingesetzten Mikroorganismen in der Regel weniger toxisch sind, können im Vergleich zu Phenol normalerweise höhere Ausbeuten erzielt werden. Hydroxybenzoesäuren können kristallisiert und aus der Fermentationsbrühe abgetrennt werden. Eine anschließende Decarboxylierung von 4-Hydroxybenzoesäure zu Phenol wurde auch bereits beschrieben. Die JP 2016-23136 A beschreibt die Reaktion unter Verwendung eines heterogenen Katalysators in Wasser als Lösungsmittel. In A.S. Lisitsyn / Applied Catalysis A: General 332 ; 2007 (166-170) wird die Decarboxylierung in Diphenylether unter Verwendung eines Kupferkatalysators beschrieben. L. J. Gooßen et al. beschreiben in ChemCatChem 2010, 2, 430-442 die Decarboxylierung mittels Silber- oder Kupferkatalysator in NMP als Lösungsmittel. Auch in Dalton Transactions (24), 4683-4688; 2009 wird die Decarboxylierung in Toluol beschrieben.

Um Phenole mit einer hohen Reinheit zu erhalten, muss das Lösungsmittel bei den im Stand der Technik beschriebenen Methoden zunächst entfernt werden. Häufig verbleiben jedoch Lösungsmittelreste im Phenol, welche den weiteren Einsatz dieses Phenols für die Herstellung beispielsweise von Monomeren wie Diarylcarbonate oder Bisphenole erschwert oder Einfluss auf die Ausbeute dieser Verfahren nimmt.

Ebenso ist auch beim Einsatz homogener Katalysatoren für die Decarboxylierung von Hydroxybenzoesäure die Abtrennbarkeit des Katalysators vom Phenol und möglichst auch das Recycling des Katalysators sicherzustellen.

Der vorliegenden Erfindung lag daher die Aufgabe zugrunde, ein Verfahren zur Herstellung von speziellen Hydroxyverbindungen der Formel (I) mittels Decarboxylierung einer Carbonsäureverbindung der Formel (II) oder eines entsprechenden Salzes dieser Carbonsäureverbindung der Formel (II) bereitzustellen, welches mindestens einen Nachteil des Stands der Technik verbessert. Insbesondere lag der vorliegenden Erfindung die Aufgabe zu Grunde ein Verfahren bereitzustellen, welches die Hydroxyverbindung der Formel (I) in hoher Reinheit liefert. Dadurch soll die erhaltene Hydroxyverbindung der Formel (I) insbesondere für die Verwendung als Edukt für weitere chemische Verbindungen geeignet sein. Insbesondere sollte die Hydroxyverbindung der Formel (I) bereitgestellt werden durch ein Verfahren, bei dem die Aufarbeitung des Produktes möglichst unaufwendig und damit vorzugsweise kostengünstig und umweltschonend ist. Dabei war es wünschenswert, dass ein heterogener Katalysator eingesetzt wird, da dadurch die Abtrennung des Katalysators von der Hydroxyverbindung der Formel (I) bereits erleichtert ist.

Mindestens eine, bevorzugt alle der oben genannten Aufgaben wurden durch die vorliegende Erfindung gelöst. Überraschend wurde gefunden, dass die Decarboxylierung einer Carbonsäureverbindung der Formel (II) oder eines entsprechenden Salzes dieser Carbonsäureverbindung der Formel (II) unter Verwendung eines heterogenen Katalysators in einer Hydroxyverbindung der Formel (I) als Lösungsmittel effektiv durchgeführt werden kann. Bevorzugt ist die Ausbeute an der gewünschten Hydroxyverbindung der Formel (I) sogar höher als unter den im Stand der Technik beschriebenen Bedingungen. Die Verwendung mindestens einer Hydroxyverbindung der Formel (I) als Lösungsmittel während der Reaktion der Decarboxylierung bietet insbesondere den Vorteil, dass eine Reaktionsmischung erhalten wird, die ohne aufwendige Aufarbeitung nach Abtrennung des Katalysators direkt als Edukt für weitere chemische Reaktionen eingesetzt werden kann. Dies liegt zum einen daran, dass die Reaktion nahezu vollständig verläuft. Zum anderen ist eine Abtrennung des Lösungsmittels nicht notwendig. Besonders bevorzugt kann das Verfahren so ausgelegt werden, dass das eingesetzte Lösungsmittel der gewünschten herzustellenden Hydroxyverbindung der Formel (I) entspricht. Dadurch wird nach vollständiger Decarboxylierung und Abtrennung des Katalysators eine nahezu reine Hydroxyverbindung der Formel (I) erhalten.

Erfindungsgemäß wird daher ein Verfahren zur Herstellung einer Hydroxyverbindung der Formel (I) bereitgestellt 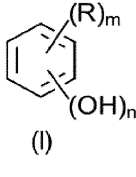 in der
R für eine lineare oder verzweigte Alkylgruppe mit 1 bis 6 Kohlenstoffatomen steht,
n 1 oder 2 ist und
m 0, 1, 2 oder 3 ist,
durch Decarboxylierung einer Carbonsäureverbindung der Formel (II) oder eines entsprechenden Salzes dieser Carbonsäureverbindung der Formel (II) 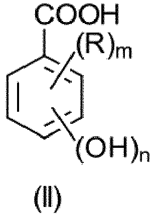
in der R, n und m die oben genannten Bedeutungen haben,
unter Verwendung mindestens eines heterogenen Katalysators,
dadurch gekennzeichnet, dass während der gesamten Reaktion der Decarboxylierung mindestens eine Hydroxyverbindung der Formel (I) im stöchiometrischen Überschuss zur Carbonsäureverbindung der Formel (II) vorhanden ist, wobei die Decarboxylierung bei einer Temperatur durchgeführt wird, die über der Schmelztemperatur sowohl der gebildeten Hydroxyverbindung der Formel (I) als auch der im stöchiometrischen Überschuss verwendeten, mindestens einen Hydroxyverbindung der Formel (I), liegt.

Gemäß der vorliegenden Erfindung liegt die Carbonsäureverbindung der Formel (II) oder das Salz der Carbonsäureverbindung der Formel (II) neben mindestens einer Hydroxyverbindung der Formel (I) vor. Dies gilt auch vor Beginn der Reaktion der Decarboxylierung. Die mindestens eine Hydroxyverbindung der Formel (I) liegt in einem stöchiometrischem Überschuss vor. Dies bedeutet, dass die Carbonsäureverbindung der Formel (II) oder das Salz der Carbonsäureverbindung der Formel (II) in einem molaren Unterschuss zur mindestens einer Hydroxyverbindung der Formel (I) vorliegt. Während der Decarboxylierung wird dann zusätzlich die Hydroxyverbindung der Formel (I) als Zielprodukt gebildet. Diese kann gleich oder verschieden, bevorzugt gleich zur mindestens einen Hydroxyverbindung zur Formel (I) sein. Erfindungsgemäß ist somit der Fall ausgeschlossen, bei dem die Hydroxyverbindung der Formel (I) sich als Zielprodukt in situ bildet und sich dann gegebenenfalls irgendwann ein molarer Unterschuss der Carbonsäureverbindung der Formel (II) oder des Salzes der Carbonsäureverbindung der Formel (II) bildet, da von Anfang der Decarboxylierung an zusätzlich mindestens eine Hydroxyverbindung der Formel (I) vorhanden sein muss. Erfindungsgemäß ist es bevorzugt, dass die Carbonsäureverbindung der Formel (II) oder das entsprechende Salz der Carbonsäureverbindung der Formel (II) in der mindestens einen Hydroxyverbindung der Formel (I), welche im stöchiometrischen Überschuss zur Carbonsäureverbindung der Formel (II) bei der gesamten Reaktion der Decarboxylierung vorhanden ist, gelöst wird, bevor die Reaktion der Decarboxylierung durchgeführt wird. Damit ist die Carbonsäureverbindung der Formel (II) oder das Salz der Carbonsäureverbindung der Formel (II) bevorzugt löslich in der mindestens einen Hydroxyverbindung der Formel (I), welche im stöchiometrischen Überschuss zur Carbonsäureverbindung der Formel (II) bei der gesamten Reaktion der Decarboxylierung vorhanden ist. Die Begriffe "lösen" und "in Lösung" sind erfindungsgemäß gemäß dem Wissen des Fachmanns zu verstehen. Bevorzugt bedeutet "lösen" und "in Lösung", wenn bei Filtration einer Flüssigkeit, in der eine Substanz gelöst ist, mit gängigen Filtermethoden kein Feststoff abgetrennt werden kann.

Das erfindungsgemäße Verfahren wird bei einer Temperatur durchgeführt, die über der Schmelztemperatur sowohl der gebildeten Hydroxyverbindung der Formel (I) als auch der im stöchiometrischen Überschuss verwendeten, mindestens einen Hydroxyverbindung der Formel (I), liegt. Damit wird das erfindungsgemäße Verfahren bevorzugt in Lösung durchgeführt. Die mindestens eine Hydroxyverbindung der Formel (I), welche im stöchiometrischen Überschuss zur Carbonsäureverbindung der Formel (II) bei der gesamten Reaktion der Decarboxylierung vorhanden ist, dient dabei als Lösungsmittel.

Bevorzugt wird das erfindungsgemäße Verfahren bei einer Temperatur von 100 bis 400 °C, besonders bevorzugt bei 150 bis 300 °C und ganz besonders bevorzugt von 160 bis 250 °C durchgeführt.

Beim erfindungsgemäßen Verfahren kann es sich um ein batch, semi-batch oder kontinuierliches Verfahren handeln.

Bevorzugt dient das erfindungsgemäße Verfahren zur Herstellung einer Hydroxyverbindung der oben gezeigten Formel (I), in der R für eine tert-Butyl-, Propyl- oder Methylgruppe steht, n 1 oder 2, bevorzugt 1 ist und m 0, 1, 2 oder 3 ist. Besonders bevorzugt dient das erfindungsgemäße Verfahren der Herstellung von 4-Propylphenol, ortho-, para- oder metha-Methylphenol (Cresole), 2,4-Dimethylphenol, 2,5-Dimethylphenol, 4-tert-Butyl-phenol oder Phenol. Ganz besonders bevorzugt ist das erfindungsgemäße Verfahren dadurch gekennzeichnet, dass die Hydroxyverbindung der Formel (I) Phenol ist.

Ebenso ist es bevorzugt, dass die mindestens eine Hydroxyverbindung der Formel (I), welche im stöchiometrischen Überschuss zur Carbonsäureverbindung der Formel (II) bei der gesamten Reaktion der Decarboxylierung vorhanden ist, eine Hydroxyverbindung der oben gezeigten Formel (I) ist, in der R für eine tert-Butyl-, Propyl- oder Methylgruppe steht, n 1 oder 2, bevorzugt 1 ist und m 0, 1, 2 oder 3 ist. Besonders bevorzugt ist diese mindestens eine Hydroxyverbindung der Formel (I) welche im stöchiometrischen Überschuss zur Carbonsäureverbindung der Formel (II) bei der gesamten Reaktion der Decarboxylierung vorhanden ist, 4-Propylphenol, ortho-, para- oder metha-Methylphenol (Cresole), 2,4-Dimethylphenol, 2,5-Dimethylphenol, 4-tert-Butyl-phenol oder Phenol. Ganz besonders bevorzugt ist sie Phenol.

Dabei ist es bevorzugt, dass die nach dem erfindungsgemäßen Verfahren hergestellte Hydroxyverbindung der Formel (I) der Hydroxyverbindung der Formel (I), welche im stöchiometrischen Überschuss zur Carbonsäureverbindung der Formel (II) bei der gesamten Reaktion der Decarboxylierung vorhanden ist, entspricht.

Erfindungsgemäß werden die Carbonsäureverbindung der Formel (II) oder das Salz der Carbonsäureverbindung der Formel (II) gelegentlich auch als Carbonsäureverbindung der Formel (II) zusammengefasst. Gemeint sind aber immer die freie Säure und/oder das Salz, soweit nicht anders angegeben. Erfindungsgemäß können auch Mischungen unterschiedlicher Carbonsäureverbindungen der Formel (II) oder unterschiedlicher Salze der Carbonsäureverbindungen der Formel (II) oder auch Mischungen mindestens einer Carbonsäureverbindung der Formel (II) mit mindestens einem Salz der Carbonsäureverbindung der Formel (II) eingesetzt werden.

Im erfindungsgemäßen Verfahren ist es bevorzugt, dass das Kation des Salzes der Carbonsäureverbindung der Formel (II) ausgewählt wird aus der Gruppe bestehend aus Alkalimetallkationen, Erdalkalimetallkationen, Ammonium, Phosphonium, Kationen von Mangan, Eisen, Kobalt, Nickel, Kupfer, Zink, Molybdän, Cadmium und beliebigen Mischungen davon. Besonders bevorzugt ist das Kation des Salzes der Carbonsäureverbindung der Formel (II) ausgewählt aus der Gruppe bestehend aus Alkalimetallkationen, Erdalkalimetallkationen und Mischungen davon.

Des Weiteren ist es erfindungsgemäß bevorzugt, dass die Carbonsäureverbindung der Formel (II) oder das entsprechende Salz der Carbonsäureverbindung der Formel (II) ausgewählt wird aus der Gruppe, bestehend aus 2-Hydroxybenzoesäure, 4-Hydroxybenzoesäure und den entsprechenden Salzen. Ganz besonders bevorzugt handelt es sich um 4-Hydroxybenzoesäure oder das entsprechende Salz.

Als Katalysator im erfindungsgemäßen Verfahren kommen grundsätzlich alle heterogenen Katalysatoren in Frage, welche bei einer Decarboxylierungsreaktion aktiv sind. Diese sind dem Fachmann bekannt. Bevorzugt wird der im erfindungsgemäßen Verfahren eingesetzte heterogene Katalysator ausgewählt aus der Gruppe, bestehend aus Al₂O₃, H₃PO₄ geträgert auf Al₂O₃, PtClₓ geträgert auf Al₂O₃, Cu/Al/Ga-MOFs, Pt-Al-MOFs, Palladium geträgert auf Aktivkohle, Platin geträgert auf Aktivkohle, Zeolithe, wie beispielsweise ZSM-5, HZSM-5, Fe₂O₃ geträgert auf MCM-41 (Mobil Composition of Matter No. 41), Fe₂O₃ geträgert auf Al-MCM-41, Pt geträgert auf SAPO-34 (Silicoaluminophosphat), Pt geträgert auf SAPO-11, Pt Hydrotalcite, Pt geträgert auf SiO₂ und beliebigen Mischungen davon. Besonders bevorzugt ist das erfindungsgemäße Verfahren dadurch gekennzeichnet, dass der mindestens eine heterogene Katalysator ein Zeolith ist. Ganz besonders bevorzugt ist das erfindungsgemäße Verfahren dadurch gekennzeichnet, dass das Zeolith eine Faujasit-Struktur aufweist.

Dem Fachmann sind Zeolithe und insbesondere Zeolithe mit Faujasit-Struktur bekannt. Die Kristallstruktur von Faujasit ist identisch mit der des synthetischen Zeolith Y. Das Grundelement des Faujasitgerüsts sind Sodalithkäfige, die über hexagonale Prismen miteinander verbunden sind. Ganz besonders bevorzugt ist der erfindungsgemäß eingesetzte Katalysator vom Zeolith Y Typ.

In einem Aspekt der Erfindung ist es des Weiteren bevorzugt, dass die Carbonsäureverbindung der Formel (II) oder das entsprechende Salz der Carbonsäureverbindung der Formel (II) durch Fermentation oder aus Zuckern, Lignocellulose, lignocellulose-haltigen Materialien, Furanen und/oder Lignin erhalten wurde. Damit ist die Carbonsäureverbindung der Formel (II) oder das entsprechende Salz der Carbonsäureverbindung der Formel (II) bevorzugt biobasiert. Im Sinne der vorliegenden Erfindung wird unter dem Ausdruck "biobasiert" verstanden, dass die betreffende chemische Verbindung zum Anmeldezeitpunkt durch einen erneuerbaren und/oder nachwachsenden Rohstoff zugänglich, erhältlich und/oder bevorzugt ein solcher erneuerbarer und/oder nachwachsender Rohstoff ist. Unter einem erneuerbaren und/oder nachwachsenden Rohstoff wird bevorzugt ein Rohstoff verstanden, welcher durch natürliche Prozesse mit einer Geschwindigkeit regeneriert wird, die mit ihrer Abbaurate vergleichbar ist (Siehe CEN/TS 16295:2012). Der Ausdruck dient insbesondere der Abgrenzung zu Rohstoffen aus fossilen Rohstoffen, erfindungsgemäß auch als petrobasiert bezeichnet. Ob ein Rohstoff biobasiert ist oder petrobasiert, kann durch die Messung von Kohlenstoffisotopen in dem Rohstoff festgestellt werden, da die relativen Mengen des Kohlenstoffisotops C14 geringer sind in fossilen Rohstoffen. Dies kann beispielsweise gemäß der ASTM D6866-18 (2018) oder der ISO16620-1 bis -5 (2015) oder der DIN SPEC 91236 2011-07 erfolgen.

Erfindungsgemäß wird der Begriff "petrobasiert" bevorzugt für solche Verbindungen verwendet, welche einen C14 Isotopengehalt von unter 0,3 × 10⁻¹², besonders bevorzugt von 0,2 × 10⁻¹² und ganz besonders bevorzugt von 0,1 × 10⁻¹² aufweist. Handelt es sich bei der Hydroxyverbindung der Formel (I) um Phenol so wird ein petrobasiertes Phenol bevorzugt über den Hock-Prozess erhalten.

Dem Fachmann ist bekannt, wie er Carbonsäureverbindung der Formel (II) oder das entsprechende Salz der Carbonsäureverbindung der Formel (II) durch Fermentation oder aus Zuckern, Lignocellulose, lignocellulose-haltigen Materialien, Furanen und/oder Lignin erhalten kann.

Dies ist beispielsweise in der WO 2015174446, WO 2015156271, US20040143867, Appl. Environ Microbiol 84 2018 :e02587-17, WO2016114668, Biomass and Bioenergy 93:209-216 October 2016, Biotechnol Bioeng. 2016 Jul;113(7):1493-503, ACS Catal., 2016, 6 (9), pp 6141-6145 oder Biotechnol. Bioeng., 113: 1493-1503, Appl Microbiol Biotechnol. 2018 Oct;102(20):8685-8705, Microbiology. 1994 Apr;140 ( Pt 4):897-904, Journal of Biotechnology 132 (2007) 49-56, WO2000018942, US 6030819, EP2698435, Bioprocess Biosyst Eng (2017) 40: 1283, US2996540, US9206449, Nature 2014, 515, 249-252, Biomass and Bioenergy 93 (2016) 209-216, 3 Biotech. 2015 Oct; 5(5): 647-651, Appl Environ Microbiol. 2018 Mar 15; 84(6): e02587-17, US3360553A beschrieben.

In diesem Aspekt der Erfindung ist es besonders vorteilhaft, dass durch den Einsatz einer biobasierten Carbonsäureverbindung der Formel (II) oder eines entsprechenden Salzes der Carbonsäureverbindung der Formel (II) eine biobasierte Hydroxyverbindung der Formel (I) erhalten wird. Diese wiederum kann zur Herstellung von weiteren biobasierten Verbindungen, beispielsweise Diarylcarbonaten, Bisphenolen oder Polycarbonaten genutzt werden wodurch letztendlich biobasierte Polymere zugänglich und auf einem effizienten und kostengünstigen Weg hergestellt werden.

Daher betrifft die vorliegende Erfindung in einem weiteren Aspekt ein Verfahren zur Herstellung eines Diarylcarbonats, eines Bisphenols oder eines Polycarbonats, dadurch gekennzeichnet, dass zur Herstellung des Diarylcarbonats, des Bisphenols oder des Polycarbonats das unmittelbare Verfahrensprodukt des erfindungsgemäßen Verfahrens in sämtlichen Bevorzugungen und bevorzugten Kombinationen nach Entfernung des heterogenen Katalysators verwendet wird. Wie bereits oben dargelegt, ermöglicht es das erfindungsgemäße Verfahren, dass nach Durchführung der Decarboxylierung eine Mischung erhalten wird, welche die hergestellte Hydroxyverbindung der Formel (I) und die mindestens eine Hydroxyverbindung der Formel (I), welche im stöchiometrischen Überschuss zur Carbonsäureverbindung der Formel (II) bei der gesamten Reaktion der Decarboxylierung vorhanden ist, umfasst. Bevorzugt können diese beiden Hydroxyverbindungen identisch sein. Wird aus dieser Mischung der Katalysator entfernt, so liegt eine im Wesentlichen reine Mischung der Hydroxyverbindungen der Formel (I) vor. Diese kann ohne weitere aufwendige Aufreinigungsschritte dem erfindungsgemäßen Verfahren zur Herstellung eines Diarylcarbonats, eines Bisphenols oder eines Polycarbonats zugeführt werden. Die Abtrennung des Katalysators ist dem Fachmann bekannt. Sie kann beispielsweise durch Filtration erzielt werden. Unter dem Ausdruck "unmittelbares Verfahrensprodukt" ist somit insbesondere zu verstehen, dass keine vorherige Abtrennung eines Lösungsmittels aus dem Verfahrensprodukt notwendig ist. Da diese Entfernung eines Lösungsmittels meist mit einer thermischen Belastung verbunden ist, wurde somit die erfindungsgemäße Mischung der Hydroxyverbindung der Formel (I) weniger thermischem Stress ausgesetzt als entsprechende Verbindungen des Stands der Technik.

Erfindungsgemäß ist es in diesem Fall besonders bevorzugt, dass es sich bei der Carbonsäureverbindung der Formel (II) oder dem Salz der Carbonsäureverbindung der Formel (II) um 4-Hydroxybenzoesäure oder das entsprechende Salz handelt.

Verfahren zur Herstellung von Diarylcarbonaten oder Bisphenolen sind dem Fachmann bekannt. Diarylcarbonate können beispielsweise durch die Reaktion der Hydroxyverbindung der Formel (I) mit einem Carbonylhalogenid, bevorzugt Phosgen, mit Kohlenstoffmonoxid oder mit Dimethylcarbonat auf bekannte Art und Weise hergestellt werden. Bisphenole können durch Reaktion der Hydroxyverbindung der Formel (I) mit einem Keton oder einem Aldehyd auf bekannte Art und Weise erhalten werden. Auch sind dem Fachmann Verfahren zur Herstellung von Polycarbonaten unter Verwendung der Hydroxyverbindung der Formel (I) bekannt. Beispielsweise kann die Hydroxyverbindung der Formel (I) als Kettenabbrecher in einem Phasengrenzflächenverfahren zur Herstellung von Polycarbonat auf bekannte Art und Weise eingesetzt werden.

Bei diesen Verfahren können die weiteren Reaktionspartner, wie beispielsweise die Ketone oder Aldehyde, ebenfalls biobasiert oder petrobasiert, bevorzugt biobasiert sein.

Des Weiteren ist es bei diesem Verfahren bevorzugt, dass die Carbonsäureverbindung der Formel (II) oder das entsprechende Salz der Carbonsäureverbindung der Formel (II) im erfindungsgemäßen Verfahren zur Herstellung einer Hydroxyverbindung der Formel (I) in sämtlichen oben beschriebenen Bevorzugungen und Kombinationen durch Fermentation oder aus Zuckern, Lignocellulose, Furanen und/oder Lignin erhalten wurde und dass die mindestens eine Hydroxyverbindung der Formel (I), welche im stöchiometrischen Überschuss zur Carbonsäureverbindung der Formel (II) bei der gesamten Reaktion der Decarboxylierung vorhanden ist, im erfindungsgemäßen Verfahren zur Herstellung einer Hydroxyverbindung der Formel (I) in sämtlichen oben beschriebenen Bevorzugungen und Kombinationen petrobasiert ist oder dass die Carbonsäureverbindung der Formel (II) oder das entsprechende Salz der Carbonsäureverbindung der Formel (II) im erfindungsgemäßen Verfahren zur Herstellung einer Hydroxyverbindung der Formel (I) in sämtlichen oben beschriebenen Bevorzugungen und Kombinationen petrobasiert ist und dass die mindestens eine Hydroxyverbindung der Formel (I), welche im stöchiometrischen Überschuss zur Carbonsäureverbindung der Formel (II) bei der gesamten Reaktion der Decarboxylierung vorhanden ist, im erfindungsgemäßen Verfahren zur Herstellung einer Hydroxyverbindung der Formel (I) in sämtlichen oben beschriebenen Bevorzugungen und Kombinationen durch Fermentation oder aus Zuckern, Lignocellulose, Furanen und/oder Lignin erhalten wurde. Dies bedeutet, dass es erfindungsgemäß bevorzugt ist, dass eine Mischung aus einer petrobasierten Hydroxyverbindung der Formel (I) mit einer biobasierten Hydroxyverbindung der Formel (I) erhalten wird. Dabei kann entweder die erfindungsgemäß hergestellte Hydroxyverbindung der Formel (I) biobasiert sein und dann die als Lösungsmittel eingesetzte Hydroxyverbindung der Formel (I) petrobasiert oder umgekehrt. Auf diese Bevorzugung wird unten als Ausführungsform 2 Bezug genommen.

Derzeit existieren unterschiedliche Kennzeichnungen, ab wann ein Produkt als "Biobasiert" bezeichnet werden darf (siehe unter anderem das Zertifizierungsprogramm "biobasierte Produkte nach ASTM D6866-18 (2018) oder der ISO16620-1 bis -5 (2015) oder der DIN SPEC 91236 2011-07 vom TÜVRheinland^{®}). Diese unterschiedlichen Kennzeichnungen setzen eine bestimmte Prozentzahl an biobasiertem Kohlenstoff im Produkt voraus. Das erfindungsgemäße Verfahren ermöglicht es, den Anteil an biobasiertem Kohlenstoff einfach einzustellen, ohne dabei das erfindungsgemäße Verfahren zu stören. Dies kann einfach durch Hinzugeben oder Austauschen der Carbonsäureverbindung der Formel (II) beziehungsweise ihres Ursprungs oder der mindestens einen Hydroxyverbindung der Formel (I), welche im stöchiometrischen Überschuss zur Carbonsäureverbindung der Formel (II) bei der gesamten Reaktion der Decarboxylierung vorhanden ist, beziehungsweise ihres Ursprungs erfolgen. Somit ermöglicht es das erfindungsgemäße Verfahren auch, in Zukunft auf Veränderungen in Kennzeichnungsanforderungen in bereits bestehenden Anlagen ohne zusätzlichen Aufwand zu reagieren. Damit kann selbst bei strengen Anforderungen immer noch eine Hydroxyverbindung der Formel (I) mit entsprechendem Label als biobasiert einfach hergestellt werden.

Ganz besonders vorteilhaft ist es, wenn das erfindungsgemäße Verfahren zur Herstellung von Diarylcarbonaten, Bisphenolen oder Polycarbonaten dadurch gekennzeichnet ist, dass die Hydroxyverbindung der Formel (I), welche bei der Reaktion zur Herstellung des Diarylcarbonats, Bisphenols, oder Polycarbonats nicht reagiert hat, vom Diarylcarbonat, Bisphenol oder Polycarbonat abgetrennt wird und dann dem erfindungsgemäßen Verfahren zur Herstellung einer Hydroxyverbindung der Formel (I) in sämtlichen oben beschriebenen Bevorzugungen und Kombinationen als Hydroxyverbindung der Formel (I), welche im stöchiometrischen Überschuss zur Carbonsäureverbindung der Formel (II) bei der gesamten Reaktion der Decarboxylierung vorhanden ist, zugeführt wird. Diese Verfahrensführung ist insgesamt besonders günstig, da die nicht reagierte Hydroxyverbindung der Formel (I) wieder als Lösungsmittel eingesetzt werden kann. Dies ist aus ökologischen und ökonomischen Gesichtspunkten vorteilhaft. Besonders bevorzugt erfolgt diese Rückführung der Hydroxyverbindung der Formel (I) beim erfindungsgemäßen Verfahren zur Herstellung von Diarylcarbonaten oder Bisphenolen.

Handelt es sich bei der nicht reagierten Hydroxyverbindung der Formel (I) um eine spezielle Mischung aus biobasierter und petrobasierter Hydroxyverbindung, kann der Fachmann entsprechende Maßnahmen im erfindungsgemäßen Verfahren zur Herstellung einer Hydroxyverbindung der Formel (I) ergreifen, um dieses Verhältnis weiter aufrecht zu erhalten. Er kann beispielsweise zusätzlich die mindestens eine Hydroxyverbindung der Formel (I), welche im stöchiometrischen Überschuss zur Carbonsäureverbindung der Formel (II) bei der gesamten Reaktion der Decarboxylierung vorhanden ist, entsprechenden Ursprungs zu der nicht reagierten Hydroxyverbindung der Formel (I) hinzudosieren.

Eine Abtrennung der nicht reagierten Hydroxyverbindung der Formel (I) vom Diarylcarbonat oder Bisphenol ist dem Fachmann bekannt. Sie kann beispielsweise über eine Destillation auf bekannte Art und Weise erfolgen.

Wie bereits oben beschrieben, weisen diese Diarylcarbonate, Bisphenole oder Polycarbonate ein speziell einstellbares Verhältnis von biobasiertem und petrobasiertem Kohlenstoff auf. Dieses ist durch das erfindungsgemäße Verfahren zugänglich. Bevorzugte Bisphenole sind solche der Formel (2a)

HO-Z-OH (2a),

in welcher
Z ein aromatischer Rest mit 6 bis 30 C-Atomen ist, der einen oder mehrere aromatische Kerne enthalten kann, substituiert sein kann und aliphatische oder cycloaliphatische Reste bzw. Alkylaryle oder Heteroatome als Brückenglieder enthalten kann.

Bevorzugt steht Z in Formel (2a) für einen Rest der Formel (3) 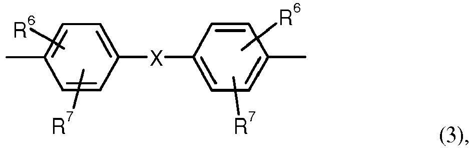 in der
R⁶ und R⁷unabhängig voneinander für H, C₁- bis C₁₈-Alkyl-, C₁- bis C₁₈-Alkoxy, Halogen wie Cl oder Br oder für jeweils gegebenenfalls substituiertes Aryl- oder Aralkyl, bevorzugt für H oder C₁- bis C₁₂-Alkyl, besonders bevorzugt für H oder C₁- bis C₈-Alkyl und ganz besonders bevorzugt für H oder Methyl stehen, und
X für eine Einfachbindung, -SO₂-, -CO-, -O-, -S-, C₁- bis C₆-Alkylen, C₂- bis C₅-Alkyliden oder C₅- bis C₆-Cycloalkyliden, welches mit C₁- bis C₆-Alkyl, vorzugsweise Methyl oder Ethyl, substituiert sein kann, ferner für C₆- bis C₁₂-Arylen, welches gegebenenfalls mit weiteren Heteroatome enthaltenden aromatischen Ringen kondensiert sein kann, steht.

Bevorzugt steht X für eine Einfachbindung, C₁- bis C₅-Alkylen, C₂- bis Cs-Alkyliden, C₅- bis C₆-Cycloalkyliden, -O-, -SO-, -CO-, -S-, -SO₂-
oder für einen Rest der Formel (3a)

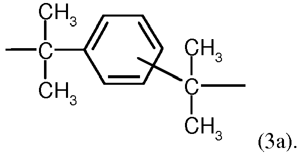

Beispiele für Bisphenole sind: Dihydroxybenzole, Dihydroxydiphenyle, Bis-(hydroxyphenyl)-alkane, Bis-(hydroxyphenyl)-cycloalkane, Bis-(hydroxyphenyl)-aryle, Bis-(hydroxyphenyl)-ether, Bis-(hydroxyphenyl)-ketone, Bis-(hydroxyphenyl)-sulfide, Bis-(hydroxyphenyl)-sulfone, Bis-(hydroxyphenyl)-sulfoxide, 1,1'-Bis-(hydroxyphenyl)-diisopropylbenzole sowie deren kernalkylierte und kernhalogenierte Verbindungen.

Bevorzugte Bisphenole sind 4,4'-Dihydroxydiphenyl, 2,2-Bis-(4-hydroxyphenyl)-1-phenylpropan, 1,1-Bis-(4-hydroxyphenyl)-phenylethan, 2,2-Bis-(4-hydroxyphenyl)propan, 2,4-Bis-(4-hydroxy-phenyl)-2-methylbutan, 1,3-Bis-[2-(4-hydroxyphenyl)-2-propyl]benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol und 1,1-Bis-(4-hydroxyphenyl)-3,3,5-trimethylcyclohexan (Bisphenol TMC).

Besonders bevorzugte Bisphenole sind 4,4'-Dihydroxydiphenyl, 1,1-Bis-(4-hydroxyphenyl)-phenyl-ethan, 2,2-Bis-(4-hydroxyphenyl)-propan, 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Bevorzugte Diarylcarbonate sind die der Formel (2) 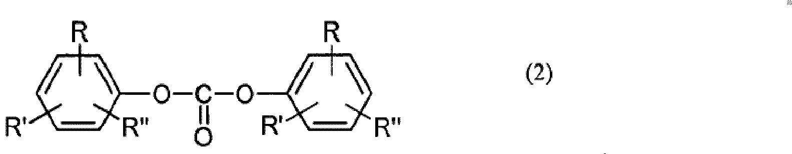 wobei R, R' und R" jeweils unabhängig voneinander gleich oder verschieden sein können und für Wasserstoff, gegebenenfalls verzweigtes C1-C34-Alkyl, C7-C34-Alkylaryl oder C6-C34-Aryl stehen, R weiterhin auch -COO-R‴ bedeuten kann, wobei R‴ für gegebenenfalls verzweigtes C1-C34-Alkyl, C7-C34-Alkylaryl oder C6-C34-Aryl steht. Solche Diarylcarbonate sind beispielsweise in EP-A 1 609 818 beschrieben. Bevorzugt sind Diphenylcarbonat, 4-tert-Butylphenyl-phenylcarbonat, Di-(4-tert-butylphenyl)-carbonat, Biphenyl-4-yl-phenyl-carbonat, Di-(biphenyl-4-yl)-carbonat, 4-(1 -Methyl-1 -phenylethyl)-phenyl-phenyl-carbonat und Di-[4-(1 -methyl-1-phenylethyl)-phenyl]-carbonat. Ganz besonders bevorzugt ist substituiertes oder unsubstituiertes, bevorzugt unsubstituiertes Diphenylcarbonat.

Des Weiteren wird ein Polycarbonat bereitgestellt, welches erhalten wird durch Polymerisation des Diarylcarbonats und / oder Bisphenols.

Solche Verfahren zur Herstellung von Polycarbonat mittels Polymersiation von Diarylcarbonaten und/oder Bisphenolen sind dem Fachmann bekannt. Beispielsweise können die Bisphenole und evtl. Verzweiger in wässriger alkalischer Lösung gelöst werden und mit einer gegebenenfalls in einem Lösemittel gelösten Carbonatquelle, insbesondere einem Carbonylhalogenid wie Phosgen, mit Kohlenstoffmonoxid oder mit Dimethylcarbonat in einem Zweiphasengemisch aus einer wässrigen alkalischen Lösung, einem organischen Lösemittel und einem Katalysator, bevorzugt einer Aminverbindung, zur Reaktion gebracht werden. Die Reaktionsführung kann auch mehrstufig erfolgen. Solche Verfahren zur Herstellung von Polycarbonat sind als Zweiphasengrenzflächenverfahren grundsätzlich z. B. aus H. Schnell, Chemistry and Physics of Polycarbonates, Polymer Reviews, Vol. 9, Interscience Publishers, New York 1964 S. 33 ff. und auf Polymer Reviews, Vol. 10, "Condensation Polymers by Interfacial and Solution Methods", Paul W. Morgan, Interscience Publishers, New York 1965, Kap. VIII, S. 325 bekannt und die grundlegenden Bedingungen daher dem Fachmann geläufig.

Alternativ können die Polycarbonate auch nach dem Schmelzumesterungsverfahren hergestellt werden. Das Schmelzumesterungsverfahren ist beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) sowie der DE-C 10 31 512 beschrieben. Beim Schmelzumesterungsverfahren werden die Bisphenole, mit Diarylcarbonaten unter Zuhilfenahme von geeigneten Katalysatoren und gegebenenfalls weiteren Zusatzstoffen in der Schmelze umgeestert.

In einem weiteren Aspekt der vorliegenden Erfindung wird ein Verfahren zur Einstellung des Isotopenverhältnisses von C14 zu C12 in einem Polymer, bevorzugt Polycarbonat bereitgestellt, dadurch gekennzeichnet, dass das Verfahren die folgenden Schritte umfasst:
(a1) Durchführung des erfindungsgemäßen Verfahren zur Herstellung einer Hydroxyverbindung der Formel (I) in sämtlichen oben beschriebenen Bevorzugungen und Kombinationen, wobei ein molares Verhältnis der Hydroxyverbindung der Formel (I), welche im stöchiometrischen Überschuss zur Carbonsäureverbindung der Formel (II) bei der gesamten Reaktion der Decarboxylierung vorhanden ist und welches petrobasiert ist, zu der Hydroxyverbindung der Formel (I) als Verfahrensprodukt des Verfahrens nach dem erfindungsgemäßen Verfahren erhalten wird,
(b1) gegebenenfalls Veränderung des molaren Verhältnisses aus Verfahrensschritt (a1) durch Hinzufügen entweder einer Hydroxyverbindung der Formel (I), welche petrobasiert ist, oder einer Hydroxyverbindung der Formel (I), welche durch Fermentation oder aus Zuckern, Lignocellulose, Furanen und/oder Lignin erhalten wurde,
(c1) Herstellung eines Diarylcarbonats oder Bisphenols unter Verwendung der nach Verfahrensschritt (b1) erhaltenen Mischung von Hydroxyverbindungen der Formel (I) und
(dl) Herstellung eines Polymers, bevorzugt Polycarbonats, unter Verwendung mindestens eines Diarylcarbonats und/oder Bisphenols des Verfahrensschritts (c1).

Wie bereits oben näher beschrieben, weiß der Fachmann, dass das Isotopenverhältnis von C14 und C12 in einem Polymer ein Indikator für die Tatsache ist, ob ein Polymer als biobasiert bezeichnet werden kann oder nicht. Methoden zur Bestimmung dieser Isotopen und damit auch des Verhältnisses wurden bereits oben beschrieben. Bevorzugt erfolgt die Bestimmung des Isotopenverhältnisses gemäß ASTM D6866-18 (2018) oder der ISO16620-1 bis -5 (2015) oder der DIN SPEC 91236 2011-07. Ebenso wird für Verfahrensschritt (c1) und auch (dl) auf die bereits oben beschriebenen bevorzugten Bisphenole, Diarylcarbonate und deren Verfahren zur Polymerisierung, um ein Polycarbonat zu erhalten, verwiesen. Auch durch dieses erfindungsgemäße Verfahren wird es ermöglicht, in Zukunft auf Veränderungen in Kennzeichnungsanforderungen in bereits bestehenden Anlagen ohne zusätzlichen Aufwand zu reagieren. Damit kann selbst bei strengen Anforderungen immer noch Polymer, bevorzugt Polycarbonat mit entsprechendem Label als biobasiert einfach hergestellt werden.

Gegenstand der vorliegenden Erfindung ist auch ein Verfahren zur Herstellung eines Bisphenols, das die folgenden Schritte umfasst:
(a2) Durchführung des erfindungsgemäßen Verfahrens zur Herstellung einer Hydroxyverbindung der Formel (I), wobei eine Hydroxyverbindung der Formel (I) erhalten wird,
(b2) Umsetzung der Hydroxyverbindung der Formel (I) aus Schritt (a2) mit einem Keton oder einem Aldehyd zu einem Bisphenol.

Gegenstand der vorliegenden Erfindung ist außerdem ein Verfahren zur Herstellung eines Diarylcarbonats, das die folgenden Schritte umfasst:
(a3) Durchführung des erfindungsgemäßen Verfahrens zur Herstellung einer Hydroxyverbindung der Formel (I), wobei eine Hydroxyverbindung der Formel (I) erhalten wird,
(b3) Umsetzung der Hydroxyverbindung der Formel (I) aus Schritt (a3) mit einem Carbonylhalogenid, mit Kohlenstoffmonoxid oder mit Dimethylcarbonat zu einem Diarylcarbonat.

Ein weiterer Gegenstand der vorliegenden Erfindung ist ein Verfahren zur Herstellung eines Polycarbonats, das die folgende Schritte umfasst:
(a4) Durchführung des erfindungsgemäßen Verfahren zur Herstellung eines Bisphenols, wobei ein Bisphenol erhalten wird,
(b4) Umsetzung des Bisphenols aus Schritt (a4) mit einem Carbonylhalogenid, mit Kohlenstoffmonoxid oder mit Dimethylcarbonat zu einem Polycarbonat.

Ein nochmals weiterer Gegenstand der vorliegenden Erfindung ist ein Verfahren zur Herstellung eines Polycarbonats, das die folgende Schritte umfasst:
(a5) Durchführung des erfindungsgemäßen Verfahrens zur Herstellung eines Diarylcarbonats, wobei ein Diarylcarbonat erhalten wird,
(b5) Umsetzung des Diarylcarbonats aus Schritt (a5) mit einem Bisphenol zu einem Polycarbonat.

Ein wiederum nochmals weiterer Gegenstand der vorliegenden Erfindung ist ein Verfahren zur Herstellung eines Polycarbonats, das die folgende Schritte umfasst:
(a6) Durchführung des erfindungsgemäßen Verfahrens zur Herstellung eines Bisphenols, wobei ein Bisphenol erhalten wird,
(b6) Durchführung des erfindungsgemäßen Verfahrens zur Herstellung eines Diarylcarbonats, wobei ein Diarylcarbonat erhalten wird,
(c6) Umsetzung des Bisphenols aus Schritt (a6) mit einem Diarylcarbonat aus Schritt (b6) zu einem Polycarbonat.

Durch all diese genannten Gegenstände wird die vorliegende Erfindung verwirklicht.

### Beispiele

### Abkürzungen:

bara: Absolutdruck in bar
U/min: Umdrehungen pro Minute
¹H-NMR: Protonenresonanzspektroskopie
M: Stoffmenegenkonzentration in mol/L
aq.: wässrige Lösung

### Chemikalien:

4-Hydroxybenzoesäure (4-HBA): Reinheit ≥ 99 %, Sigma-Aldrich Chemie GmbH
VE-Wasser (H₂O): vollentsalztes Wasser aus Ringleitung
Natriumhydroxid (NaOH): wasserfrei, Reinheit ≥ 97 %, Sigma-Aldrich Chemie GmbH
aq. NaOH-Lösung aus VE-Wasser und Natriumhydroxid hergestellt
Phenol: Reinheit ≥ 96 %, Sigma-Aldrich Chemie GmbH
Hexadeuterodimethylsulfoxid (DMSO-d6): Reinheit ≥ 96 %, Euriso-Top GmbH

### Katalysatoren:

CBV 600 (CAS 1318-02-1), Zeolyst International, Inc., Oberfläche 660 m²/g, Porengröße 2.43 nm, Si/Al-Verhältnis 2.5. Der Katalysator wurde vor Verwendung 3 h bei 300 °C in Luft kalziniert.

Faujasite (Produktreferenz: BCR704), Sigma-Aldrich Chemie GmbH, Oberfläche 567 m²/g, Porengröße 0.67 nm, Si/Al-Verhältnis 1.6. Der Katalysator wurde wie erhalten verwendet.

### Allgemeine Versuchsvorschrift - Versuche mit Katalysator:

0,5 g 4-Hydroxybenzoesäure, 0,5 mL Lösungsmittel (siehe Tabelle) und 0,02 g des jeweiligen Katalysators (siehe Tabelle) wurden in einem 10 mL-Druckreaktor vorgelegt, mit Argon als Schutzgas gespült und der Reaktor verschlossen. Anschließend wurde ein Argon-Druck von 3 bara beaufschlagt, 10 min bei 800 U/min gerührt und der Druck auf 1,5 bara abgelassen. Dieser Vorgang wurde noch einmal wiederholt, bevor der Reaktor auf die Reaktionstemperatur von 230 °C gebracht wurde. Nach der entsprechenden Reaktionszeit (siehe Tabelle) bei dieser Temperatur wurde der Druckreaktor auf Raumtemperatur abgekühlt und der Druck abgelassen. Das erhaltene Reaktionsgemisch wurde in Ethanol aufgenommen, der feste Katalysator wurde mittels Zentrifugation (5 min, 5000 U/min, Hettich Universal 320) abgetrennt und die Lösung am Rotationsverdampfer von Ethanol befreit. Das so isolierte Reaktionsprodukt wurde anschließend mittels ¹H-NMR untersucht.

### Allgemeine Versuchsvorschrift - Versuche ohne Katalysator:

Die Vorgehensweise war analog zur allgemeinen Versuchsvorschrift für Versuche mit Katalysator. Es wurde lediglich ohne Katalysator gearbeitet.

### ¹H-NMR zur Bestimmung von 4-Hydroxybenzoesäure und Phenol im Reaktionsprodukt:

Etwa 100 mg des gewonnenen Reaktionsprodukts wurden in 0,5 mL DMSO-d6 gelöst und ein ¹H-NMR-Spektrum auf einem Bruker Avance 400 bei 400 MHz vermessen. Die Auswertung der erhaltenen Spektren erfolgte anhand der untenstehenden spezifischen Verschiebungen und Integrale.

| 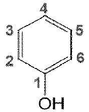 | 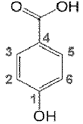 |
|---|---|
| Phenol - ¹H-NMR (400 MHz, DMSO-d6): δ (ppm) 9.4 (C_{OH}, 1H), 7.10-7.20 (C_{3,5}, 2H), 6.72-6.78 (C_{2,4,6}, 3H) | 4-Hydroxybenzoesäure - ¹H-NMR (400 MHz, DMSO-d6): δ (ppm) 12.3 (C_{OOH}, 1H) 10.1 (C_{OH}, 1H), 7.8 (C_{3,5}, 2H), 6.80-6.86 (C_{2,6}, 2H) |

**Tabelle 1:**

| # | Katalysator | Lösungsmittel | Reaktionszeit [h] | Reaktionsprodukt [molares Verhältnis aus ¹H-NMR] |
|---|---|---|---|---|
| 1 | Faujasite | H₂O | 2 | 2:1 Phenol/4-Hydroxybenzoesäure |
| 2 | Faujasite | Phenol | 2 | Phenol |
| 3 | CBV 600 | H₂O | 2 | 2:1 Phenol/4-Hydroxybenzoesäure |
| 4 | CBV 600 | Phenol | 2 | 3:1 Phenol/4-Hydroxybenzoesäure^{a} |
| 5 | - | H₂O | 2 | 1:1 Phenol/4-Hydroxybenzoesäure |
| a) Die Angabe des molaren Verhältnisses bezieht sich nur auf gebildetes Phenol. | | | | |

Wie der Tabelle zu entnehmen ist, kann bei ansonsten gleichen Bedingungen durch die Verwendung einer Hydroxyverbindung der Formel (I) (in den Beispielen Phenol) der Umsatz zum gewünschten Produkt im Verhältnis zu Wasser als Lösungsmittel erhöht werden. Dies gilt für unterschiedliche heterogene Katalysatoren.

## Patentansprüche

1. Verfahren zur Herstellung einer Hydroxyverbindung der Formel (I) 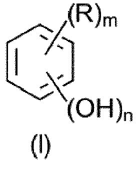 in der
R für eine lineare oder verzweigte Alkylgruppe mit 1 bis 6 Kohlenstoffatomen steht,
n 1 oder 2 ist und
m 0, 1,2 oder 3 ist,
durch Decarboxylierung einer Carbonsäureverbindung der Formel (II) oder eines entsprechenden Salzes dieser Carbonsäureverbindung der Formel (II) 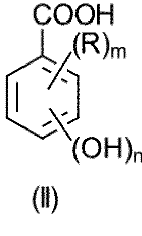
in der R, n und m die oben genannten Bedeutungen haben,
unter Verwendung mindestens eines heterogenen Katalysators,
**dadurch gekennzeichnet, dass** während der gesamten Reaktion der Decarboxylierung mindestens eine Hydroxyverbindung der Formel (I) im stöchiometrischen Überschuss zur Carbonsäureverbindung der Formel (II) vorhanden ist, wobei die Decarboxylierung bei einer Temperatur durchgeführt wird, die über der Schmelztemperatur sowohl der gebildeten Hydroxyverbindung der Formel (I) als auch der im stöchiometrischen Überschuss verwendeten, mindestens einen Hydroxyverbindung der Formel (I), liegt.

2. Verfahren nach Anspruch 1, **dadurch gekennzeichnet, dass** die Carbonsäureverbindung der Formel (II) oder das entsprechende Salz der Carbonsäureverbindung der Formel (II) in der mindestens einen Hydroxyverbindung der Formel (I), welche im stöchiometrischen Überschuss zur Carbonsäureverbindung der Formel (II) bei der gesamten Reaktion der Decarboxylierung vorhanden ist, gelöst wird bevor die Reaktion der Decarboxylierung durchgeführt wird.

3. Verfahren nach Anspruch 1 oder 2, **dadurch gekennzeichnet, dass** der mindestens eine heterogene Katalysator ein Zeolith ist.

4. Verfahren nach Anspruch 3, **dadurch gekennzeichnet, dass** das Zeolith eine Faujasit-Struktur aufweist.

5. Verfahren nach einem der Ansprüche 1 bis 4, **dadurch gekennzeichnet, dass** das Kation des Salzes der Carbonsäureverbindung der Formel (II) ausgewählt wird aus der Gruppe bestehend aus Alkalimetallkationen, Erdalkalimetallkationen, Ammonium, Phosphonium, Kationen von Mangan, Eisen, Kobalt, Nickel, Kupfer, Zink, Molybdän, Cadmium und beliebigen Mischungen davon.

6. Verfahren nach einem der Ansprüche 1 bis 5, **dadurch gekennzeichnet, dass** die Carbonsäureverbindung der Formel (II) oder das entsprechende Salz der Carbonsäureverbindung der Formel (II) durch Fermentation oder aus Zuckern, Lignocellulose, lignocellulose-haltigen Materialien, Furanen und/oder Lignin erhalten wurde.

7. Verfahren nach einem der Ansprüche 1 bis 6, **dadurch gekennzeichnet, dass** die hergestellte Hydroxyverbindung der Formel (I) der Hydroxyverbindung der Formel (I), welche im stöchiometrischen Überschuss zur Carbonsäureverbindung der Formel (II) bei der gesamten Reaktion der Decarboxylierung vorhanden ist, entspricht.

8. Verfahren nach einem der Ansprüche 1 bis 7, **dadurch gekennzeichnet, dass** die Hydroxyverbindung der Formel (I) Phenol ist.

9. Verfahren nach einem der Ansprüche 1 bis 8, **dadurch gekennzeichnet, dass** die Carbonsäureverbindung der Formel (II) oder das entsprechende Salz der Carbonsäureverbindung der Formel (II) ausgewählt wird aus der Gruppe, bestehend aus 2-Hydroxybenzoesäure, 4-Hydroxybenzoesäure und den entsprechenden Salzen.

10. Verfahren zur Einstellung des Isotopenverhältnisses von C14 zu C12 in einem Polymer, bevorzugt Polycarbonat, **dadurch gekennzeichnet, dass** das Verfahren die folgenden Schritte umfasst:
(a1) Durchführung des Verfahrens nach einem der Ansprüche 1 bis 9, wobei ein molares Verhältnis der Hydroxyverbindung der Formel (I), welche im stöchiometrischen Überschuss zur Carbonsäureverbindung der Formel (II) bei der gesamten Reaktion der Decarboxylierung vorhanden ist und welches petrobasiert ist, zu der Hydroxyverbindung der Formel (I) als Verfahrensprodukt des Verfahrens nach einem der Ansprüche 1 bis 9 erhalten wird,
(b1) gegebenenfalls Veränderung des molaren Verhältnisses aus Verfahrensschritt (a1) durch Hinzufügen entweder einer Hydroxyverbindung der Formel (I), welche petrobasiert ist, oder einer Hydroxyverbindung der Formel (I), welche durch Fermentation oder aus Zuckern, Lignocellulose, Furanen und/oder Lignin erhalten wurde,
(c1) Herstellung eines Diarylcarbonats oder Bisphenols unter Verwendung der nach Verfahrensschritt (b1) erhaltenen Mischung von Hydroxyverbindungen der Formel (I) und
(dl) Herstellung eines Polymers, bevorzugt Polycarbonats, unter Verwendung mindestens eines Diarylcarbonats und/oder Bisphenols des Verfahrensschritts (c1).

11. Verfahren zur Herstellung eines Bisphenols, **dadurch gekennzeichnet, dass** das Verfahren die folgenden Schritte umfasst:
(a2) Durchführung des Verfahrens nach einem der Ansprüche 1 bis 9 zur Herstellung einer Hydroxyverbindung der Formel (I), wobei eine Hydroxyverbindung der Formel (I) erhalten wird,
(b2) Umsetzung der Hydroxyverbindung der Formel (I) aus Schritt (a2) mit einem Keton oder einem Aldehyd zu einem Bisphenol.

12. Verfahren zur Herstellung eines Diarylcarbonats, wobei das Verfahren folgende Schritte umfasst:
(a3) Durchführung des Verfahrens nach einem der Ansprüche 1 bis 9 zur Herstellung einer Hydroxyverbindung der Formel (I), wobei eine Hydroxyverbindung der Formel (I) erhalten wird,
(b3) Umsetzung der Hydroxyverbindung der Formel (I) aus Schritt (a3) mit einem Carbonylhalogenid, mit Kohlenstoffmonoxid oder mit Dimethylcarbonat zu einem Diarylcarbonat.

13. Verfahren zur Herstellung eines Polycarbonats, wobei das Verfahren folgende Schritte umfasst:
(a4) Durchführung des Verfahrens nach Anspruch 11 zur Herstellung eines Bisphenols, wobei ein Bisphenol erhalten wird,
(b4) Umsetzung des Bisphenols aus Schritt (a4) mit einem Carbonylhalogenid, mit Kohlenstoffmonoxid oder mit Dimethylcarbonat zu einem Polycarbonat.

14. Verfahren zur Herstellung eines Polycarbonats, wobei das Verfahren folgende Schritte umfasst:
(a5) Durchführung des Verfahrens nach Anspruch 12 zur Herstellung eines Diarylcarbonats, wobei ein Diarylcarbonat erhalten wird,
(b5) Umsetzung des Diarylcarbonats aus Schritt (a5) mit einem Bisphenol zu einem Polycarbonat.

15. Verfahren zur Herstellung eines Polycarbonats, wobei das Verfahren folgende Schritte umfasst:
(a6) Durchführung des Verfahrens nach Anspruch 11 zur Herstellung eines Bisphenols, wobei ein Bisphenol erhalten wird,
(b6) Durchführung des Verfahrens nach Anspruch 12 zur Herstellung eines Diarylcarbonats, wobei ein Diarylcarbonat erhalten wird,
(c6) Umsetzung des Bisphenols aus Schritt (a6) mit einem Diarylcarbonat aus Schritt (b6) zu einem Polycarbonat.

## Claims

1. Process for producing a hydroxy compound of the formula (I) 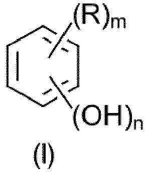 in which
R is a linear or branched alkyl group having 1 to 6 carbon atoms,
n is 1 or 2, and
m is 0, 1, 2, or 3,
by decarboxylation of a carboxylic acid compound of the formula (II) or of a corresponding salt of said carboxylic acid compound of the formula (II) 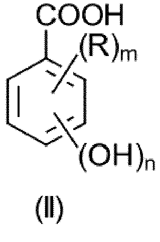
in which R, n, and m are as defined above,
using at least one heterogeneous catalyst,
**characterized in that** at least one hydroxy compound of the formula (I) is throughout the decarboxylation reaction present in a stoichiometric excess relative to the carboxylic acid compound of the formula (II), the decarboxylation being carried out at a temperature that is above the melting temperature both of the hydroxy compound of the formula (I) that is formed and of the at least one hydroxy compound of the formula (I) used in a stoichiometric excess.

2. Process according to Claim 1, **characterized in that**, before carrying out the decarboxylation reaction, the carboxylic acid compound of the formula (II) or corresponding salt of the carboxylic acid compound of the formula (II) is dissolved in the at least one hydroxy compound of the formula (I) that throughout the decarboxylation reaction is present in a stoichiometric excess relative to the carboxylic acid compound of the formula (II).

3. Process according to Claim 1 or 2, **characterized in that** the at least one heterogeneous catalyst is a zeolite.

4. Process according to Claim 3, **characterized in that** the zeolite has a faujasite structure.

5. Process according to any of Claims 1 to 4, **characterized in that** the cation of the salt of the carboxylic acid compound of the formula (II) is selected from the group consisting of alkali metal cations, alkaline earth metal cations, ammonium, phosphonium, cations of manganese, iron, cobalt, nickel, copper, zinc, molybdenum, cadmium, and any desired mixtures thereof.

6. Process according to any of Claims 1 to 5, **characterized in that** the carboxylic acid compound of the formula (II) or corresponding salt of the carboxylic acid compound of the formula (II) was obtained by fermentation or from sugars, lignocellulose, lignocellulose-containing materials, furans, and/or lignin.

7. Process according to any of Claims 1 to 6, **characterized in that** the produced hydroxy compound of the formula (I) corresponds to the hydroxy compound of the formula (I) that throughout the decarboxylation reaction is present in a stoichiometric excess relative to the carboxylic acid compound of the formula (II).

8. Process according to any of Claims 1 to 7, **characterized in that** the hydroxy compound of the formula (I) is phenol.

9. Process according to any of Claims 1 to 8, **characterized in that** the carboxylic acid compound of the formula (II) or corresponding salt of the carboxylic acid compound of the formula (II) is selected from the group consisting of 2-hydroxybenzoic acid, 4-hydroxybenzoic acid, and the corresponding salts.

10. Process for adjusting the C14 to C12 isotope ratio in a polymer, preferably polycarbonate, **characterized in that** the process comprises the following steps:
(a1) executing the process according to any of Claims 1 to 9, wherein a molar ratio of the hydroxy compound of the formula (I) that throughout the decarboxylation reaction is present in a stoichiometric excess relative to the carboxylic acid compound of the formula (II), and that is petroleum-based, to the hydroxy compound of the formula (I) as process product of the process according to any of Claims 1 to 9 is maintained,
(b1) optionally altering the molar ratio from process step (a1) through the addition either of a hydroxy compound of the formula (I) that is petroleum-based or of a hydroxy compound of the formula (I) obtained by fermentation or from sugars, lignocellulose, furans, and/or lignin,
(c1) producing a diaryl carbonate or bisphenol using the mixture of hydroxy compounds of the formula (I) obtained in process step (b1), and
(d1) producing a polymer, preferably polycarbonate, using at least one diaryl carbonate and/or bisphenol from process step (c1).

11. Process for producing a bisphenol, **characterized in that** the process comprises the following steps:
(a2) executing the process according to any of Claims 1 to 9 for the production of a hydroxy compound of the formula (I), wherein a hydroxy compound of the formula (I) is obtained,
(b2) reacting the hydroxy compound of the formula (I) from step (a2) with a ketone or an aldehyde to afford a bisphenol.

12. Process for producing a diaryl carbonate, wherein the process comprises the following steps:
(a3) executing the process according to any of Claims 1 to 9 for the production of a hydroxy compound of the formula (I), wherein a hydroxy compound of the formula (I) is obtained,
(b3) reacting the hydroxy compound of the formula (I) from step (a3) with a carbonyl halide, with carbon monoxide or with dimethyl carbonate to afford a diaryl carbonate.

13. Process for producing a polycarbonate, wherein the process comprises the following steps:
(a4) executing the process according to Claim 11 for the production of a bisphenol, wherein a bisphenol is obtained,
(b4) reacting the bisphenol from step (a4) with a carbonyl halide, with carbon monoxide or with dimethyl carbonate to afford a polycarbonate.

14. Process for producing a polycarbonate, wherein the process comprises the following steps:
(a5) executing the process according to Claim 12 for the production of a diaryl carbonate, wherein a diaryl carbonate is obtained,
(b5) reacting the diaryl carbonate from step (a5) with a bisphenol to afford a polycarbonate.

15. Process for producing a polycarbonate, wherein the process comprises the following steps:
(a6) executing the process according to Claim 11 for the production of a bisphenol, wherein a bisphenol is obtained,
(b6) executing the process according to Claim 12 for the production of a diaryl carbonate, wherein a diaryl carbonate is obtained,
(c6) reacting the bisphenol from step (a6) with a diaryl carbonate from step (b6) to afford a polycarbonate.

## Revendications

1. Procédé de préparation d'un composé hydroxylé de Formule (I) 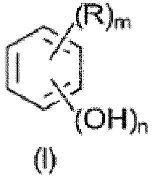 dans laquelle
R représente un groupe alkyle linéaire ou ramifié ayant 1 à 6 atomes de carbone,
n représente 1 ou 2 et
m représente 0, 1, 2 ou 3,
par décarboxylation d'un composé acide carboxylique de Formule (II) ou d'un sel correspondant de ce composé acide carboxylique de Formule (II) 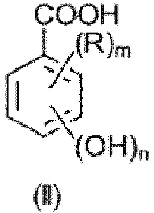
dans laquelle R, n et m ont les significations données ci-dessus,
par utilisation d'au moins un catalyseur hétérogène,
**caractérisé en ce que**, pendant la totalité de la réaction de décarboxylation, au moins un composé hydroxylé de Formule (I) est présent selon un excès stœchiométrique par rapport au composé acide carboxylique de Formule (II), la décarboxylation étant mise en œuvre à une température qui est supérieure à la température de fusion tant du composé hydroxylé formé de Formule (I) que de l'au moins un composé hydroxylé de Formule (I) utilisé selon un excès stœchiométrique.

2. Procédé selon la revendication 1, **caractérisé en ce que** le composé acide carboxylique de Formule (II) ou le sel correspondant du composé acide carboxylique de Formule (II) est dissous dans l'au moins un composé hydroxylé de Formule (I), qui est présent selon un excès stœchiométrique par rapport au composé acide carboxylique de Formule (II) pendant la totalité de la réaction de décarboxylation, et ce, avant mise en œuvre de la réaction de décarboxylation.

3. Procédé selon la revendication 1 ou 2, **caractérisé en ce que** l'au moins un catalyseur hétérogène est une zéolithe.

4. Procédé selon la revendication 3, **caractérisé en ce que** la zéolithe présente une structure de faujasite.

5. Procédé selon l'une des revendications 1 à 4, **caractérisé en ce que** le cation du sel du composé acide carboxylique de Formule (II) est choisi dans le groupe consistant en les cations de métal alcalin, les cations de métal alcalino-terreux, l'ammonium, le phosphonium, les cations de manganèse, de fer, de cobalt, de nickel, de cuivre, de zinc, de molybdène, de cadmium et tous mélanges de ceux-ci.

6. Procédé selon l'une des revendications 1 à 5, **caractérisé en ce que** le composé acide carboxylique de Formule (II) ou le sel correspondant du composé acide carboxylique de Formule (II) a été obtenu par fermentation ou à partir de sucres, de lignocellulose, de matériaux contenant de la lignocellulose, de furanes et/ou de lignine.

7. Procédé selon l'une des revendications 1 à 6, **caractérisé en ce que** le composé hydroxylé préparé de Formule (I) correspond au composé hydroxylé de Formule (I) qui est présent selon un excès stœchiométrique par rapport au composé acide carboxylique de Formule (II) pendant la totalité de la réaction de décarboxylation.

8. Procédé selon l'une des revendications 1 à 7, **caractérisé en ce que** le composé hydroxylé de Formule (I) est le phénol.

9. Procédé selon l'une des revendications 1 à 8, **caractérisé en ce que** le composé acide carboxylique de Formule (II) ou le sel correspondant du composé acide carboxylique de Formule (II) est choisi dans le groupe consistant en l'acide 2-hydroxybenzoïque, l'acide 4-hydroxybenzoïque et les sels correspondants.

10. Procédé pour ajuster le rapport isotopique C14/C12 dans un polymère, de préférence un polycarbonate, **caractérisé en ce que** le procédé comprend les étapes suivantes :
(a1) mise en œuvre du procédé selon l'une des revendications 1 à 9, grâce auquel on obtient un rapport en moles du composé hydroxylé de Formule (I), qui est présent pendant la totalité de la réaction de décarboxylation selon un excès stœchiométrique par rapport au composé acide carboxylique de Formule (II) et qui est pétrosourcé, au composé hydroxylé de Formule (I) en tant que produit du procédé selon l'une des revendications 1 à 9,
(b1) éventuellement, modification du rapport en moles de l'étape (a1) par addition soit d'un composé hydroxylé de Formule (I) qui est pétrosourcé, soit d'un composé hydroxylé de Formule (I) qui a été obtenu par fermentation ou à partir de sucres, de lignocellulose, de furanes et/ou de lignine,
(c1) préparation d'un carbonate de diaryle ou d'un bisphénol par utilisation du mélange de composés hydroxylés de Formule (I) obtenu après l'étape (b1) et
(d1) préparation d'un polymère, de préférence un polycarbonate, par utilisation d'au moins un carbonate de diaryle et/ou d'un bisphénol de l'étape (c1).

11. Procédé de préparation d'un bisphénol, **caractérisé en ce que** le procédé comprend les étapes suivantes :
(a2) mise en œuvre du procédé selon l'une des revendications 1 à 9 pour préparer un composé hydroxylé de Formule (I), avec obtention d'un composé hydroxylé de Formule (I),
(b2) réaction du composé hydroxylé de Formule (I) de l'étape (a2) avec une cétone ou un aldéhyde pour obtenir un bisphénol.

12. Procédé de préparation d'un carbonate de diaryle, le procédé comprenant les étapes suivantes :
(a3) mise en œuvre du procédé selon l'une des revendications 1 à 9 pour préparer un composé hydroxylé de Formule (I), avec obtention d'un composé hydroxylé de Formule (I),
(b3) réaction du composé hydroxylé de Formule (I) de l'étape (a3) avec un halogénure de carbonyle, avec du monoxyde de carbone ou avec du carbonate de diméthyle pour obtenir un carbonate de diaryle.

13. Procédé de préparation d'un polycarbonate, le procédé comprenant les étapes suivantes :
(a4) mise en œuvre du procédé selon la revendication 11 pour préparer un bisphénol, avec obtention d'un bisphénol,
(b4) réaction du bisphénol de l'étape (a4) avec un halogénure de carbonyle, avec du monoxyde de carbone ou avec du carbonate de diméthyle pour obtenir un polycarbonate.

14. Procédé de préparation d'un polycarbonate, le procédé comprenant les étapes suivantes :
(a5) mise en œuvre du procédé selon la revendication 12 pour préparer un carbonate de diaryle, avec obtention d'un carbonate de diaryle,
(b5) réaction du carbonate de diaryle de l'étape (a5) avec un bisphénol pour obtenir un polycarbonate.

15. Procédé de préparation d'un polycarbonate, le procédé comprenant les étapes suivantes :
(a6) mise en œuvre du procédé selon la revendication 11 pour préparer un bisphénol, avec obtention d'un bisphénol,
(b6) mise en œuvre du procédé selon la revendication 12 pour préparer un carbonate de diaryle, avec obtention d'un carbonate de diaryle,
(c6) réaction du bisphénol de l'étape (a6) avec un carbonate de diaryle de l'étape (b6) pour obtenir un polycarbonate.
